# FASCICULE DE BREVET EUROPEEN

(11) **EP 0 562 956 B1**
(45) Date de publication et mention de la délivrance du brevet: **27.09.1995**
(21) Numéro de dépôt: 93400757.6
(22) Date de dépôt: 24.03.1993
(51) Int. Cl.: C07C 233/58, A61K 31/16, C07C 233/60, C07D 207/267, C07C 275/24, C07C 233/05, A61K 31/17, A61K 31/40

(54) **Nouvelles naphtylalkylamines leur procédé de préparation et les compositions pharmaceutiques qui les contiennent**
Naphtylalkylamine, Verfahren zu ihrer Herstellung und diese enthaltende pharmazeutische Zusammensetzungen
Naphtylalkylamines, process for their preparation and pharmaceutical compositions containing them

(30) Priorité: 27.03.1992 FR 9203700
(43) Date de publication de la demande: 29.09.1993
(73) Titulaire: ADIR ET COMPAGNIE, 92415 Courbevoie Cédex (FR)
(72) Inventeur: Yous, Said, Laboratoire de Chimie Synthèse, F-59045 Lille Cédex (FR); Lesieur, Daniel, F-59147 Gondecourt (FR); Depreux, Patrick, F-59280 Armentieres (FR); Guardiola-Lemaitre, Béatrice, F-92200 Neuilly sur Seine (FR); Adam, Gérard, F-78600 Le Mesnil le Roi (FR); Renard, Pierre, F-78000 Versailles (FR); Caignard, Daniel-Henri, F-75015 Paris (FR)

(56) Documents cités:
- EP-A- 0 344 425
- EP-A- 0 447 285
- DE-A- 3 828 566
- US-A- 4 916 223
- JOURNAL OF HETEROCYCLIC CHEMISTRY. vol. 2, no. 4, Décembre 1965, PROVO US pages 379 - 354 W.R. SCHLEIGH ET AL. 'AZASTEROIDS II.APPROACHES TO THE 13- AZAEQUILENIN SYSTEM (1,2)'

## Description

L'invention concerne de nouvelles naphtylalkylamines, leur procédé de préparation et les compositions pharmaceutiques qui les contiennent.

On connait dans la littérature le 1-[2-(acétylamino)éthyl]naphtalène utilisé dans la synthèse de dérivés 1-[2-(phénylsulfonamido)éthyl] naphtalèniques présentés comme antagonistes du thromboxane (DE 3828566) et la N-[2-(napht-1-yl)éthyl] 4-bromobutyramide décrite en tant qu'intermédiaire de synthèse dans Journal of Heterocyclic Chemistry vol. 2 (4), pp 378-384 (1965).

On connait également de l'art antérieur des naphtylalkylamides de formule (a), revendiqués dans la demande EP 447285, 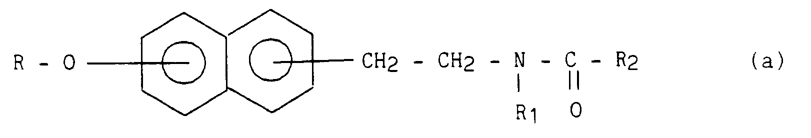
en tant qu'agonistes des récepteurs de la mélatonine.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Or la demanderesse a présentement découvert de nouvelles naphtyl alkylamines montrant une très haute affinité pour les récepteurs mélatoninergiques et possédant de façon surprenante, in vitro et in vivo, des propriétés antagonistes des récepteurs de la mélatonine très puissantes, donc contraires à celles des composés de la demande EP 447285.

Plus spécifiquement l'invention concerne les composés de formule générale (I) : 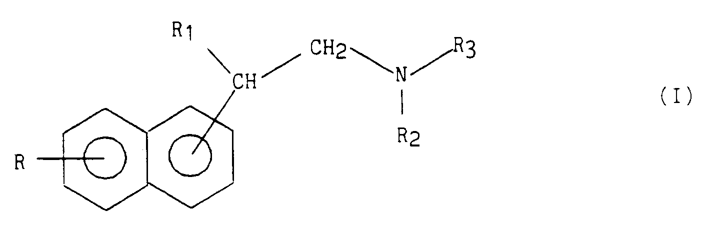
dans laquelle :
- R: représente un atome d'hydrogène ou un groupement -O-R₄ dans lequel R₄ signifie un atome d'hydrogène ou un groupement, substitué ou non substitué, choisi parmi alkyle, cycloalkyle, cycloalkylalkyle, phényle, phénylalkyle, et diphénylalkyle,
- R₁: représente un atome d'hydrogène ou un groupement -CO-O-R₅ dans lequel R₅ signifie un atome d'hydrogène ou un groupement alkyle substitué ou non substitué,
- R₂: représente un atome d'hydrogène ou un groupement -R'₂ avec R'₂ représentant un radical alkyle ou alkyle substitué,
- R₃: représente :
- un groupement 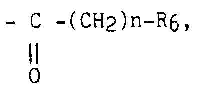 dans lequel n représente 0 ou un nombre entier de 1 à 3, et R₆ représente un atome d'hydrogène, ou un groupement alkyle, alkyle substitué, alcène, alcène substitué, cycloalkyle, cycloalkyle substitué, ou un groupement hétérocyclique substitué ou non substitué, choisi parmi : pyrrolidine, pipéridine, pipérazine, homopipéridine, homopipérazine, morpholine, et thiomorpholine ;
- un groupement 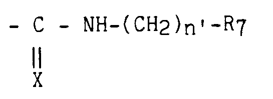 dans lequel X représente un atome d'oxygène ou de soufre, n' représente 0 ou un nombre entier de 1 à 3, et R₇ représente un groupement alkyle, alkyle substitué, cycloalkyle, cycloalkyle substitué, phényle, ou phényle substitué,
étant entendu que si :
- R représente un groupement alcoxy,
- R représente un atome d'hydrogène et R₃ représente un groupement -CO-R₈, dans lequel R₈ représente un atome d'hydrogène, un groupement méthyle, ou un groupement méthyle ou propyle substitué par un halogène,
- ou si R₃ représente un groupement 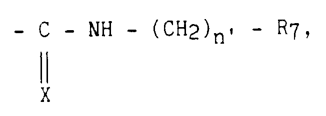 dans lequel X, n', et R₇ sont tels que définis précédemment,
alors R₁ ne peut pas être un atome d'hydrogène,
leurs isomères optiques et leurs sels d'addition à une base pharmaceutiquement acceptable,
étant entendu que, sauf précisions contraires,
- le terme "substitué" signifie que les groupements auxquels il se rapporte peuvent être substitués par un ou plusieurs radicaux choisis parmi halogène, alkyle (C₁-C₄), alcoxy (C₁-C₄), phényle, et phénylalkyle, les noyaux phényles pouvant eux-mêmes être substitués par un ou plusieurs radicaux halogène, alkyle (C₁-C₄), alcoxy (C₁-C₄), hydroxyle, ou trifluorométhyle,
- le terme "alkyle" signifie un groupement comportant de 1 à 6 atomes de carbone en chaîne droite ou ramifiée,
- le terme "alcène" signifie un groupement comportant de 2 à 6 atomes de carbone en chaine droite ou ramifiée,
- le terme "cycloalkyle" signifie un groupement contenant de 3 à 10 atomes de carbone, mono- ou bi-cyclique, saturé ou insaturé.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

L'invention s'étend également au procédé de préparation des composés de formule (I) caractérisé en ce que :
on fait réagir un composé de formule (II) : 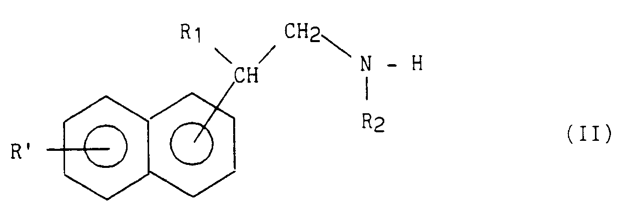
dans laquelle R₁ et R₂ sont tels que définis dans la formule (I) et R' représente un atome d'hydrogène, un groupement hydroxyle, ou un groupement alcoxy,
- soit
   . avec un halogénure d'acide de formule (III) 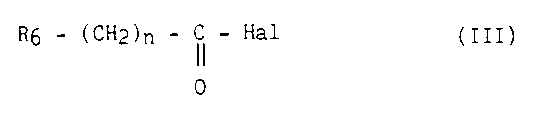 dans laquelle R₆ et n sont tels que définis dans la formule (I) et Hal représente un atome d'halogène,
   . ou avec un composé de formule (III/a), lors d'une hydrogénation catalytique, 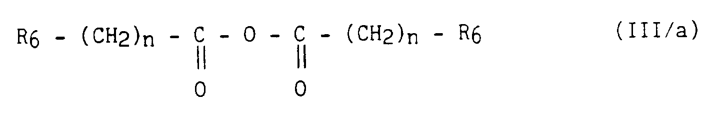
dans laquelle R₆ et n sont tels que définis prédédemment, pour obtenir un composé de formule (I/a) 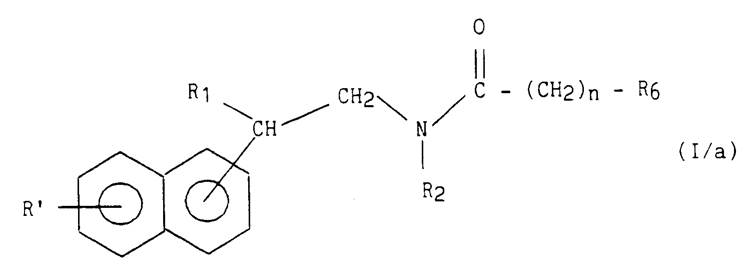
dans laquelle R', R₁, R₂, R₆, et n sont tels que définis précédemment, cas particulier des composés de formule (I) dans lesquels R représente un groupement R' et R₃ représente un groupement 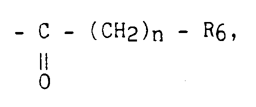
- soit avec un isocyanate ou un isothiocyanate de formule (IV) :

   X = C = N - (CH₂)n' - R₇ (IV)

   dans laquelle X, n', et R₇ sont tels que définis dans la formule (I) pour obtenir un composé de formule (I/c) : 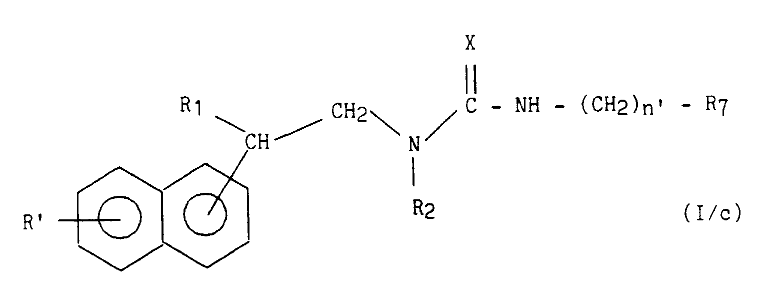
dans laquelle R', R₁, R₂, R₇, X, et n' sont tels que définis précédemment, cas particulier des composés de formules (I) dans lesquels R représente un groupement R' et R₃ représente un groupement 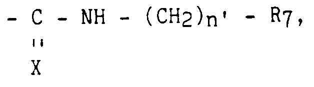
composés de formules (I/a), (I/b), et (I/c) qui peuvent être également, lorsque R' représente un groupement hydroxyle, mis en réaction avec un composé de formule (V)

R'' - Hal' (V)

dans laquelle R'' représente un groupement, non substitué ou substitué (le terme substitué étant tel que défini dans la formule I), choisi parmi cycloalkyle, cycloakylalkyle, phényle, phénylalkyle, et diphénylalkyle, et Hal' représente un atome d'halogène afin d'obtenir un composé de formule (I/d) 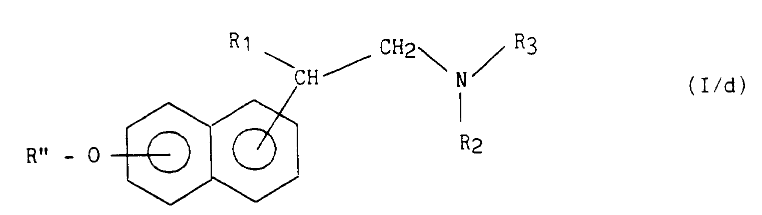
dans laquelle R₁, R₂, R₃, et R'' sont tels que définis précédemment, cas particulier des composés de formule (I) dans lesquels R représente un groupement -O- R'',
les composés de formules (I/a), (I/b), (I/c), et (I/d) forment l'ensemble des composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration , et le passage sur charbon ou résine,
- séparés, le cas échéant, sous forme pure ou sous forme de mélange, en leurs éventuels isomères optiques,
- ou salifiés, dans le cas où R₁ représente un groupement carboxyle ou dans le cas où R représente un groupement hydroxyle, par une base pharmaceutiquement acceptable.

L'invention s'étend également au procédé d'obtention des composés de formule (I/e) 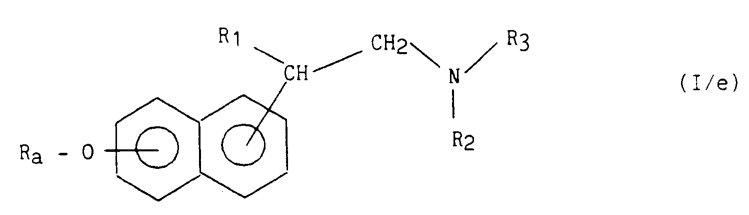
dans laquelle R₁, R₂, et R₃ sont tels que définis dans la formule (I) et Rₐ représente un groupement, saturé, cycloalkyle ou cycloalkylalkyle, cas particulier des composés de formule (I) dans lequel R représente un groupement -O-Rₐ,
caractérisé en ce que l'on soumet un composé de formule (I/f) 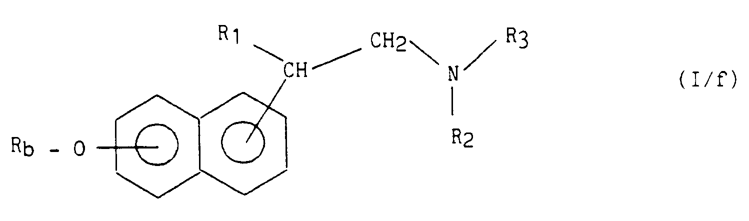
dans laquelle R₁, R₂, et R₃ sont tels que précédemment et R_{b} représente un groupement, insaturé, cycloalcényle ou cycloalcénylalkyle,
cas particulier des composés de formule (I) dans lesquels R représente un groupement - O - R_{b},
à une hydrogénation catalytique pour obtenir le composé de formule (I/a) où Rₐ correspond à la forme saturée du groupement R_{b},
les composés de formule (I/e) pouvant être :
- purifiés suivant une ou plusieurs méthodes de purification choisies parmi la cristallisation, la chromatographie sur gel de silice, l'extraction, la filtration , et le passage sur charbon ou résine,
- séparés, le cas échéant, sous forme pure ou sous forme de mélange, en leurs éventuels isomères optiques,
- ou salifiés, dans le cas où R₁ représente un groupement carboxyle, par une base pharmaceutiquement acceptable.

Les composés de formule (II) sont aisément accessibles à l'homme de l'art :
- soit, lorsque R₁ représente un groupement -CO-O-R₅ où R₅ est tel que défini dans la formule (I), par réaction d'un composé de formule (b) 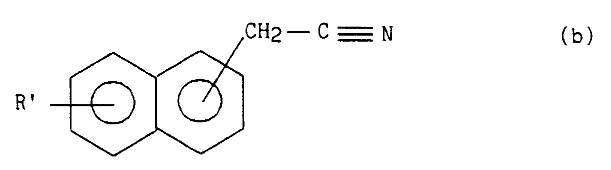
dans laquelle R' représente un atome d'hydrogène, un groupement hydroxyle, ou un groupement alcoxy, avec un composé de formule (c) 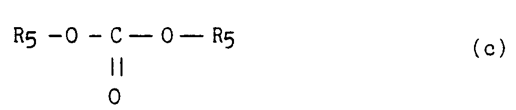
dans laquelle R₅ est tel que défini dans la formule (I) pour obtenir un composé de formule (d) 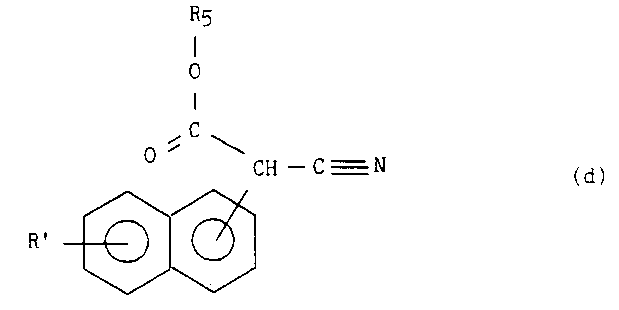
dans laquelle R' et R₅ sont tels que définis précédemment, qui est ensuite hydrogéné pour obtenir un composé de formule (II/a) 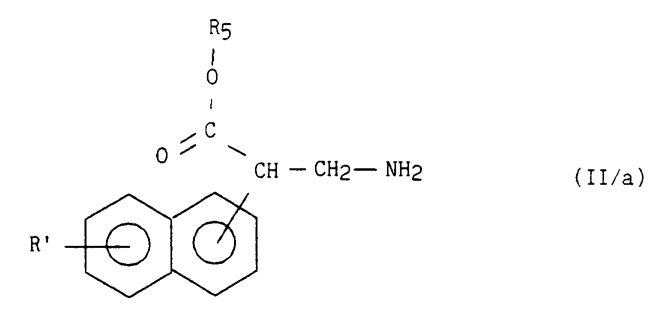
dans laquelle R' et R₅ sont tels que défini précédemment,
qui peut ensuite réagir avec un composé de formule (e)

R'₂ - Hal'' (e)

dans laquelle R'₂ est tel que défini dans la formule (I) et Hal'' représente un atome d'halogène, afin d'obtenir les composés de formule (II/b) 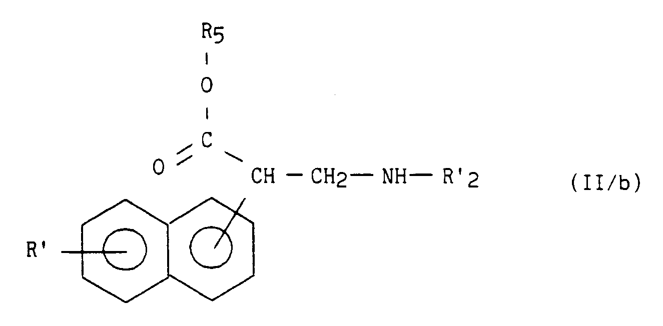
dans laquelle R', R'₂, et R₅ sont tels que définis précédemment,
- soit,lorsque R₁ représente un atome d'hydrogène, par réaction des composés de formule (II/c)

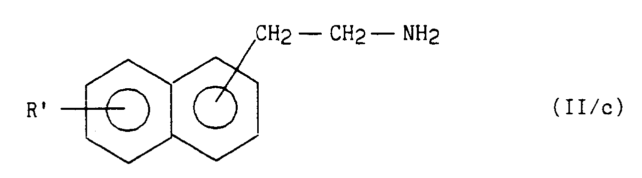

dans laquelle R' est tel que défini précédemment, avec un composé de formule (e) tel que défini précédemment,
pour obtenir des composés de formule (II/d) 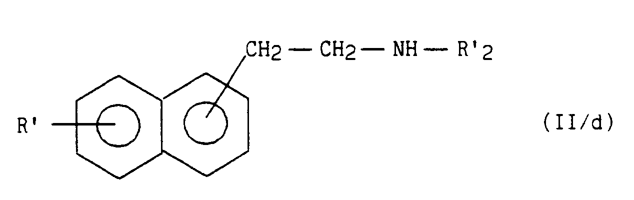
dans laquelle R' et R'₂ sont tels que défini précédemment,
les composés de formules (II/a), (II/b), (II/c) et (II/d) formant l'ensemble des composés de formule (II),
les composés de formules (II/a), (II/b), (II/c), et (II/d) pouvant être, si on le désire, purifiés, ou séparés en leurs différents isomères, ou salifiés, le cas échéant, par une base ou un acide.

Les matières premières utilisées dans les procédés précédemment décrits sont :
- soit commerciales,
- soit aisément accessibles à l'homme du métier selon des procédés décrits dans la littérature et notamment dans la demande EP 447285.

Les composés de formule (I) possèdent des propriétés pharmacologiques très intéressantes.

La demanderesse a découvert que les composés de l'invention possédaient une haute affinité sélective pour les récepteurs mélatoninergiques et antagonisaient de façon très importante l'action de la mélatonine.

Ce caractère antagoniste des composés de l'invention est mis en évidence dans l'étude pharmacologique (inhibition de la synthèse d'AMPc dans les cellules de la pars tubéralis, exemple C, et inhibition de l'activité de la mélatonine chez le xénope, exemple E) de la présente demande, et s'avère surprenant au vu de l'activité opposée (c'est à dire agoniste des récepteurs de la mélatonine) des composés les plus proches de l'art antérieur, décrits dans la demande EP 447285.

Les composés de l'invention sont donc suceptibles d'être utilisés dans le traitement des troubles liés à une activité anormale de la mélatonine dans un organisme.

Il apparaît que les composés de l'invention peuvent être également utilisés pour le traitement des troubles du système nerveux central, du système immunitaire, et du système endocrinien.

L'invention s'étend aussi aux compositions pharmaceutiques renfermant comme principe actif au moins un des composés de formule (I) ou un de ses sels d'addition avec une base pharmaceutiquement acceptable, en association avec un ou plusieurs excipients, agents liants, agents aromatisants, agents de délitement, agents édulcorants, agents lubrifiants, ou véhicules, tous convenables pour l'usage pharmaceutique.

Parmi les compositions selon l'invention, on pourra citer, à titre d'exemples et de façon non limitative, celles qui conviennent pour l'administration orale, parentérale, oculaire, per ou trans-cutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, les gels.

Les préparations ainsi obtenues se présentent généralement sous forme dosée et peuvent contenir selon les affections traitées, l'âge, et le sexe du malade, de 0,01 à 10 mg en prises de une à trois fois par jour.

Les exemples qui suivent illustrent l'invention et ne la limitent en aucune façon.

### EXEMPLE 1 : N-[2-(NAPHT-1-YL)ETHYL] CYCLOBUTANECARBOXAMIDE

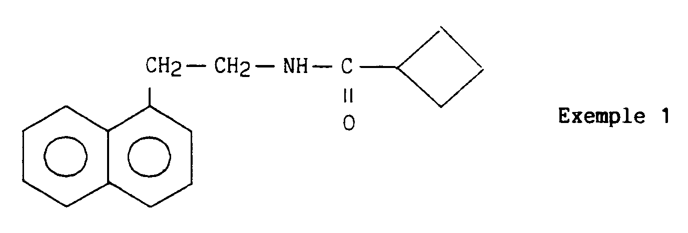

On dissout 0,01 mole du chlorhydrate de N-[2-(napht-1-yl)éthyl]amine dans un mélange eau/chloroforme (40/60 ; vol/vol) puis on ajoute sous agitation magnétique 0,02 mole de carbonate de potassium. On refroidit le mélange réactionnel dans un bain de glace, puis on ajoute goutte à goutte et sous agitation magnétique 0,01 mole du chlorure de l'acide cyclobutane carboxylique.

Après maintien de l'agitation pendant 30 minutes à température ambiante, on sépare la phase chloroformique, on la lave à l'eau, on la sèche sur chlorure de calcium puis on évapore le chloroforme sous vide pour obtenir, après recristallisation du résidu dans un mélange toluène-cyclohexane, le N-[2-(napht-1-yl)éthyl] cyclobutanecarboxamide.
Rendement : 89 %
Point de fusion : 88-89° C

| Microanalyse élémentaire : | | | |
|---|---|---|---|
| | % C | % H | % N |
| calculé | 80,59 | 7,56 | 5,52 |
| trouvé | 80,30 | 7,68 | 5,60 |

Caractéristiques spectrales
- Infrarouge :: ν NH : 3280 cm⁻¹
ν CO : 1630 cm⁻¹
RMN (CDCl₃) :
1,72 - 2,50 ppm ; (massif, 6H) ; CH₂ cyclobutyl
2,68 - 3,04 ppm ; (massif, 1H) ; CH cyclobutyl
3,28 ppm ; (triplet 2H) ; CH₂ - CH₂ - NH
3,60 ppm ; (quintuplet, 2H) ; CH₂ - CH₂ - NH
5,50 ppm ; (signal, 1H) ; NH
7,28 - 8,20 ppm ; (massif, 7H) ; H aromatiques

### EXEMPLE 2 : N-[2-(NAPHT-1-YL)ETHYL] CYCLOPROPANECARBOXAMIDE

En suivant le procédé de l'exemple 1 mais en remplaçant le chlorure de l'acide cyclobutanecarboxylique par le chlorure de l'acide cyclopropanecarboxylique, on obtient le N-[2-(napht-1-yl)éthyl] cyclopropanecarboxamide.
Solvant de recristallisation : toluène-cyclohexane
Rendement : 88 %
Point de fusion : 117-118° C

| Microanalyse élémentaire : | | | |
|---|---|---|---|
| | % C | % H | % N |
| calculé | 80,30 | 7,16 | 5,85 |
| trouvé | 80,24 | 6,89 | 5,87 |

Caractéristiques spectrales
- Infrarouge :: ν NH : 3240 cm⁻¹
ν CO : 1630 cm⁻¹
RMN (CDCl₃) :
0,50 - 1,40 ppm ; (massif, 5H) ; H cyclopropyl
3,28 ppm ; (triplet 2H) ; CH₂ - CH₂ NH
5,75 ppm ; (signal, 1H) ; NH
7,3 - 8,2 ppm ; (massif, 7H) ; H aromatiques

### EXEMPLE 3 : N-[2-(NAPHT-1-YL)ETHYL] TRIFLUOROACETAMIDE

En suivant le procédé de l'exemple 1 mais en remplaçant le chlorure de l'acide cyclobutanecarboxylique par le chlorure de trifluoroacétyle, on obtient le N-[2-(napht-1-yl)éthyl] trifluoroacétamide.
Solvant de recristallisation : cyclohexane
Rendement : 89 %
Point de fusion : 79-80 ° C

| Microanalyse élémentaire : | | | |
|---|---|---|---|
| | % C | % H | % N |
| calculé | 80,59 | 7,56 | 5,52 |
| trouvé | 80,30 | 7,68 | 5,60 |

Caractéristiques spectrales
- Infrarouge :: ν NH : 3280 cm⁻¹
ν CO : 1630 cm⁻¹
RMN (CDCl₃) :
3,30 ppm ; (triplet 2H) ; CH₂ - CH₂ - NH
3,72 ppm ; (quintuplet, 2H) ; CH₂ - CH₂ - NH
6,50 ppm ; (signal, 1H) ; NH
7,32 - 8,16 ppm ; (massif, 7H) ; H aromatiques

### EXEMPLES 4 A 10

En procédant comme dans l'exemple 1 mais en remplaçant le chlorure de l'acide cyclobutanecarboxylique par l'halogénure d'acyle approprié, on obtient successivement les composés des exemples suivants :
- **EXEMPLE 4**: **: N-[2-(NAPHT-1-YL)ETHYL] PROPIONAMIDE**
- **EXEMPLE 5**: **: N-[2-(NAPHT-1-YL)ETHYL] ISOBUTANECARBOXAMIDE**
- **EXEMPLE 6**: **: N-[2-(NAPHT-1-YL)ETHYL] CYCLOHEXANECARBOXAMIDE**
- **EXEMPLE 7**: **: N-[2-(NAPHT-1-YL)ETHYL] 2-(PIPERAZIN-1-YL)ACETAMIDE**
- **EXEMPLE 8**: **: N-[2-(NAPHT-1-YL)ETHYL] 2-(4-METHYLPIPERAZIN-1-YL)ACETAMIDE**
- **EXEMPLE 9**: **: N-[2-(NAPHT-1-YL)ETHYL] 3-CYCLOPENTYLPROPIONAMIDE**
- **EXEMPLE 10**: **: N-[2-(NAPHT-1-YL)ETHYL] BUTYRAMIDE**

### EXEMPLE 11 : N-[2-(NAPHT-1-YL) 2-(METHOXYCARBONYL)ETHYL] CYCLOBUTANECARBOXAMIDE

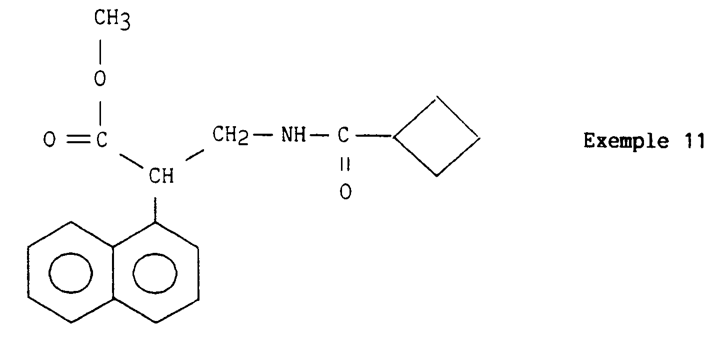

### STADE A : 2-(NAPHT-1-YL) 2-(METHOXYCARBONYL)ACETONITRILE

On dissout 0,1 mole de 2-(napht-1-yl)acétonitrile dans 120 cm³ de carbonate de diméthyle et on chauffe la solution à reflux.On ajoute ensuite 0,1 mole de sodium par petites portions durant 30 min,temps pendant lequel le méthanol s'évapore. On maintient le milieu réactionnel à reflux pendant 1 heure puis on évapore à sec l'excès de carbonate de diméthyle. On reprend le résidu par 100 cm³ d'une solution d'acide acétique à 18 % dans l'eau.

Après extraction par de l'acétate d'éthyle, la phase organique est séchée sur sulfate de magnésium puis évaporée à sec pour obtenir après recristallisation dans un mélange toluène-cyclohexane, le 2-(napht-1-yl) 2-(méthoxycarbonyl)acétonitrile.
Rendement : 88 %
Point de fusion : 89-90° C
Caractéristiques spectrales
- Infrarouge :: ν C ≡ N : 2240 cm⁻¹
ν CO (ester) : 1745 cm⁻¹
RMN (CDCl₃) :
3,78 ppm ; (singulet, 3H) ; COOCH₃
5,40 ppm ; (singulet, 1H) ; CH
7,40 - 8,70 ppm ; (massif, 7H) ; H aromatiques

### STADE B : 2-(NAPHT-1-YL) 2-(METHOXYCARBONYL)ETHYLAMINE

On dissout 0,02 mole de 2-(napht-1-yl) 2-(méthoxycarbonyl)acéto nitrile dans 250 cm³ de méthanol puis on ajoute 0,04 mole de chlorure de cobalt. On ajoute par petites fractions, en refroidissant, 0,14 mole de borohydrure de sodium, ce qui provoque la formation d'un précipité noir. On laisse agiter pendant 2 heures à température ambiante. On acidifie ensuite légèrement par addition d'acide chlorhydrique 3N jusqu'à dissolution du complexe noir puis on évapore le méthanol et on réalise une extraction avec de l'acétate d'éthyle. La phase aqueuse est alcalinisée par une solution concentrée d'ammoniac. On extrait ensuite à deux reprises par de l'acétate d'éthyle, on sèche la phase organique sur sulfate de magnésium et on porte à sec puis on reprend le résidu par de l'alcool absolu et on fait barboter de l'acide chlorhydrique gazeux. On porte à sec, et on obtient, après recristallisation dans l'acétonitrile, la 2-(napht-1-yl) 2-(méthoxycarbonyl)éthylamine.
Rendement : 42 %,
Point de fusion (chlorhydrate) : 217-219° C
Caractéristiques spectrales (chlorhydrate) :
- Infrarouge :: νNH₂ : 3300 - 2500 cm⁻¹
ν CO (ester) : 1720 cm⁻¹
RMN (DMSO) :
3,00 - 3,80 ppm ; (multiplet, 4H) ; CH₂ + H₂O
3,60 ppm ; (singulet, 3H) ; COOCH₃
5,07 ppm ; (doublet, 1H) ; CH
7,30 - 8,25 ppm ; (massif, 7H) ; H aromatiques
8,12 ppm ; (signal, 3H) ; NH₃⁺ Cl⁻

### STADE C : N-[2-(NAPHT-1-YL) 2-(METHOXYCARBONYL)ETHTL] CYCLOBUTANECARBOXAMIDE

On dissout 0,01 mole du chlorhydrate de l'amine obtenue au stade précédent dans 100 cm³ d'un mélange eau/chloroforme (40/60, vol/vol) puis on ajoute sous agitation magnétique 0,02 mole de carbonate de potassium. On refroidit le mélange réactionnel dans un bain de glace, puis on ajoute goutte à goutte et sous agitation magnétique 0,01 mole de chlorure de l'acide cyclobutanecarboxylique. Après maintien de l'agitation pendant 30 minutes à température ambiante, on sépare la phase chloroformique, on la lave à l'eau, on la sèche sur chlorure de calcium puis on évapore le chloroforme sous vide pour obtenir, après recristallisation du résidu dans un mélange toluène-cyclohexane le N-[2-(napht-1-yl) 2-(méthoxycarbonyl)éthyl]cyclobutanecarboxamide.
Rendement : 79 %
Point de fusion : 88-90° C

| Microanalyse élémentaire : | | | |
|---|---|---|---|
| | % C | % H | % N |
| calculé | 73,28 | 6,79 | 4,49 |
| trouvé | 73,19 | 6,78 | 4,47 |

Caractéristiques spectrales
- Infrarouge :: ν NH : 2240 cm⁻¹
ν CO (ester) : 1730 cm⁻¹
ν CO (amide) : 1640 cm⁻¹
RMN (CDCl₃) :
1,74 - 2,4 ppm ; (massif, 6H) ; CH₂ cyclobutyl
2,75 - 3,10 ppm ; (massif, 1H) ; CH cyclobutyl
3,40 - 4,10 ppm ; (massif, 2H) ; CH₂
3,72 ppm ; (singulet, 3H) ; COOCH₃
4,80 ppm ; (double doublet, 1H) ; CH
5,85 ppm ; (signal, 1H) ; NH
7,3 - 8,4 ppm ; (massif, 7H) ; H aromatiques

### EXEMPLE 12 : N-[2-(NAPHT-1-YL) 2-(METHOXYCARBONYL)ETHYL] CYCLOPROPANECARBOXAMIDE

En procédant comme dans l'exemple 13 mais en remplaçant au stade C le chlorure de l'acide cyclobutanecarboxylique par le chlorure de l'acide cyclopropanecarboxylique, on obtient le N-[2-(napht-1-yl)-2-(méthoxycarbonyl)éthyl] cyclopropylcarboxamide.
Solvant de recristallisation : toluène - cyclohexane
Rendement : 83 %
Point de fusion : 140-142° C

### EXEMPLES 13 ET 14

En procédant comme dans l'exemple 11, mais en remplaçant au stade C le chlorure de l'acide cyclobutanecarboxylique par l'halogénure d'acyle correspondant, on obtient les composés des exemples suivants :
- **EXEMPLE 13**: **: N-[2-(NAPHT-1-YL) 2-(METHOXYCARBONYL)ETHYL]PROPIONAMIDE**
- **EXEMPLE 14**: **: N-[2-(NAPHT-1-YL) 2-(METHOXYCARBONYL)ETHYL] 3-CYCLOPENTYL PROPIONAMIDE**

### EXEMPLES 15 A 19

En procédant comme dans l'exemple 11, mais en remplaçant au stade A le 2-(napht-1-yl)acétonitrile par le 2-(7-méthoxynapht-1-yl)acétonitrile (EP 447285) et en utilisant au stade C les halogénures d'acyle appropriés, on obtient les composés suivants :
- **EXEMPLE 15**: **: N-[2-(7-METHOXYNAPHT-1-YL) 2-(METHOXYCARBONYL)ETHYL] CYCLOPROPANECARBOXAMIDE**

Solvant de recristallisation : mélange toluène-cyclohexane
Rendement : 76 %
Point de fusion : 95-96° C

| Microanalyse élémentaire : | | | |
|---|---|---|---|
| | % C | % H | % N |
| calculé | 69,70 | 6,46 | 4,27 |
| trouvé | 70,05 | 6,53 | 4,32 |

- **EXEMPLE 16 :**: **N-[2-(7-METHOXYNAPHT-1-YL) 2-(METHOXYCARBONYL)ETHYL] CYCLOBUTANECARBOXAMIDE**
- **EXEMPLE 17 :**: **N-[2-(7-METHOXYNAPHT-1-YL) 2-(METHOXYCARBONYL)ETHYL] TRIFLUOROMETHYLCARBOXAMIDE**
- **EXEMPLE 18**: **: N-[2-(7-METHOXYNAPHT-1-YL) 2-(METHOXYCARBONYL)ETHYL] PROPIONAMIDE**
- **EXEMPLE 19**: **: N-[2-(7-METHOXYNAPHT-1-YL) 2-(METHOXYCARBONYL)ETHYL] MORPHOLINOACETAMIDE**

### EXEMPLE 20 : N-[2-(7-METHOXYNAPHT-1-YL) 2-(METHOXYCARBONYL)ETHYL] ACETAMIDE

Une solution de 0,04 mole de 2-(7-méthoxynapht-1-yl)acétonitrile dans 150 cm³ d'anhydride acétique en présence de 5 g de catalyseur (Nickel de Raney) est soumise à une pression d'hydrogène de 20,7 bars pendant 30 heures à la température de 60°C sous agitation.
Après filtration du catalyseur et évaporation du solvant sous vide, l'huile résiduelle est reprise par 250 cm³ d'acétate d'éthyle. On lave avec une solution saturée de carbonate de sodium, puis avec de l'eau. Après séchage sur sulfate de sodium, la phase organique est évaporée à sec, puis et le résidu est recristallisé dans un mélange toluène-hexane (2-1) pour obtenir le N-[2-(7-méthoxynapht-1-yl) 2-(méthoxycarbonyl)éthyl] acétamide.
Rendement : 70 %
Point de fusion : 118-120° C
Caractéristiques spectrales :
- Infrarouge :: ν NH : 3280 cm⁻¹
ν CO (ester) : 1730 cm⁻¹
ν CO (amide) : 1650 cm⁻¹

### EXEMPLES 21 A 23

En procédant comme dans l'exemple 13, mais en remplaçant au stade A le carbonate de diméthyle par le carbonate de diéthyle, on obtient les composés des exemples suivants :
- **EXEMPLE 21**: **: N-[2-(NAPHT-1-YL) 2-(ETHOXYCARBONYL)ETHYL] CYCLOBUTANE CARBOXAMIDE**
- **EXEMPLE 22**: **: N-[2-(NAPHT-1-YL) 2-(ETHOXYCARBONYL)ETHYL] CYCLOPROPANE CARBOXAMIDE**
- **EXEMPLE 23**: **: N-[2-(NAPHT-1-YL) 2-(ETHOXYCARBONYL)ETHYL] PROPIONAMIDE**

### EXEMPLE 24 : N-[2-(7-HYDROXYNAPHT-1-YL)ETHYL] CYCLOPROPANECARBOXAMIDE

Dans un ballon rodé de 250 cm³, on introduit 13,8 g (5,81.10⁻² mole) de chlorhydrate de 2-(7-méthoxynapht-1-yl) éthylamine et 46 cm³ d'une solution d'acide bromydrique à 47 %. Le mélange est porté à reflux durant 6,5 heures. Après refroidissement, le milieu réactionnel est filtré. Ce précipité est lavé à l'eau puis à l'hexane. La recristallisation du produit brut est réalisée dans un mélange acétate d'éthyle-hexane pour obtenir le bromhydrate de 2-(7-hydroxynapht-1-yl)éthylamine (rendement : 80 % ; point de fusion : 174 - 175°C).

Puis dans une fiole conique de 500 cm³, dissoudre 12,4 g (8,9.10⁻² mole) de carbonate de potassium dans 50 cm³ d'eau. Sous bonne agitation, ajouter 12,4 g (4,62.10⁻² mole) du bromhydrate de la 2-(7-hydroxynapht-1-yl) éthylamine obtenu précédemment. Additionner 200 cm³ de chloroforme, puis verser goutte à goutte une solution chloroformique (10 cm³) de 4,95 g (4,64.10⁻² mole) de chlorure de l'acide cyclopropanecarboxylique. Cette addition s'effectue sous une forte agitation. Le terme de la réaction est atteint lorsqu'il n'y a plus de solide en suspension. Décanter le milieu réactionnel, séparer la phase aqueuse, laver la phase chloroformique à l'eau puis la sécher sur sulfate de magnésium. Evaporer le solvant sous pression réduite. Après recristallisation dans le toluène, on obtient le N-[2-(7-hydroxynapht-1-yl)éthyl] cyclopropylcarboxamide.
Rendement : 67 %
F = 140 - 141°C
Caractéristiques spectrales :
- Infrarouge :: ν OH et NH : 3330 cm⁻¹
ν C=C + C-C(cyclopropyl) : 3200 - 3100 cm⁻¹
ν CH : 2900 - 3000 cm⁻¹
ν CO (amide) : 1640 cm⁻¹

### EXEMPLE 25 : N-{2-[7-(CYCLOHEXENE-3-YL)OXYNAPHT-1-YL]ETHYL} CYCLO PROPANECARBOXAMIDE

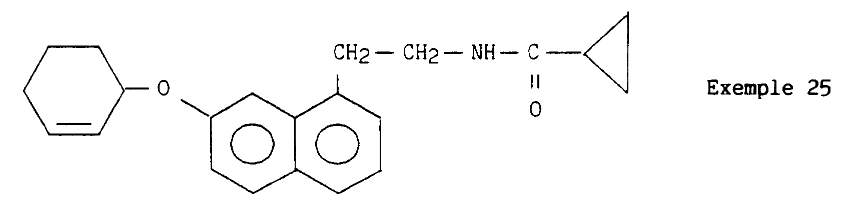

Dans un ballon rodé de 50 cm³, introduire 2,74 g (1,98.10⁻² mole) de carbonate de potassium, 3,4 g (1,33.10⁻² mole) de N-[2-(7-hydroxynapht-1-yl)éthyl] cyclopropanecarboxamide obtenu à l'exemple 24 dissout dans 20 cm³ d'acétone anhydre, et 3,4 g (2,1.10⁻² mole) de 3-bromocyclohexène. Chauffer à reflux pendant 22 heures. Le milieu réactionnel est filtré et le filtrat évaporé sous pression réduite. La recristallisation du résidu d'évaporation dans l'acétate d'éthyle permet d'obtenir le N-{2-[7-(cyclohexène-3-yl)oxynaptht-1-yl]éthyl} cyclopropanecarboxamide purifié.
Rendement : 85 %
F = 132 - 133°C

| Microanalyse élémentaire : | | | |
|---|---|---|---|
| calculé | C % 78,78 | H % 7,50 | N % 4,18 |
| trouvé | C % 78,25 | H % 7,48 | N % 4,15 |

Caractéristiques spectrales :
- Infrarouge :: ν NH (torsion) : 3300 cm⁻¹
ν C=C + C-C(cyclopropyl) : 3020 - 3150 cm⁻¹
ν CH : 2900 - 2960 cm⁻¹
ν CO (amide) : 1640 cm⁻¹
ν NH (élongation) : 1250 cm⁻¹

### EXEMPLE 26 : N-[2-(7-BENZYLOXYNAPHT-1-YL)ETHYL] CYCLOPROPANECARBOXAMIDE

Dans un ballon de 150 cm³ contenant 50 cm³ d'éthanol absolu, ajouter sous agitation magnétique et par petites fractions 0,23 gramme de sodium.

Ajouter ensuite 0,01 mole de N-[2-(7-hydroxynapht-1-yl)éthyl] cyclopropanecarboxamide (obtenue à l'exemple 24), poursuivre l'agitation pendant 30 min, puis évaporer à sec.

Le dérivé sodé obtenu est dissous dans 30 cm³ de diméthyl formamide anhydre. Sous agitation magnétique, ajouter 0,011 mole de bromure de benzyle par l'intermédiaire d'une ampoule à brome.

Chauffer à 90°C pendant 4 heures. Laisser refroidir, puis reverser le milieu réactionnel sur de la glace. Essorer la précipité formé, laver avec une solution d'hydroxyde de sodium 1N puis à l'eau. Sécher et recristalliser pour obtenir le N-[2-(7-benzyloxynapht-1-yl)éthyl] cyclopropanecarboxamide purifié.
Solvant de recristallisation : mélange alcool à 95° - eau
Rendement : 66 %
Point de fusion : 119-120°C
Caractéristiques spectrales :
- Infrarouge :: ν NH : 3280 cm⁻¹
ν CO : 1625 cm⁻¹

### EXEMPLE 27 : N-[2-(7-DIPHENYLMETHYLOXYNAPHT-1-YL)ETHYL] CYCLOPROPANECARBOXAMIDE

En procédant comme dans l'exemple 26 mais en remplaçant le bromure de benzyle par le bromure de (diphényl)méthyle, on obtient le N-[2-(7-diphénylméthyloxynapht-1-yl)éthyl]cyclopropanecarboxamide.
Solvant de recristallisation : hexane
Rendement : 62 %
Point de fusion : 109-111°C
Caractéristiques spectrales :
- Infrarouge :: ν NH : 3280 cm⁻¹
ν CO : 1635 cm⁻¹

### EXEMPLE 28 : N-[2-(7-CYCLOHEXYLOXYNAPHT-1-YL)ETHYL] CYCLOPROPANECARBOXAMIDE

Une solution alcoolique (35 cm³) de 0,8 g (2,385.10⁻³ mole) du composé cyclohexénique obtenu à l'exemple 25 est soumis à une hydrogènation catalytique à froid, durant 15 heures sous une pression d'hydrogène de 1 bar, en présence de 0,8 g de palladium activé sur charbon. Le milieu réactionnel est ensuite filtré, puis le filtrat est évaporé sous pression réduite. Le résidu d'évaporation est recristallisé dans un mélange méthanol-eau. On obtient 0,65 g de produit purifié du N-[2-(7-cyclohexyloxynapht-1-yl)éthyl] cyclopropylcarboxamide.
Rendement : 81 %
Solvant de recristallisation : méthanol - eau
Point de fusion : 153-155°C
Caractéristiques spectrales :
- Infrarouge :: ν NH (torsion) : 3300 cm⁻¹
ν C = C + C - C (cyclopropyl) : 3020 - 3150 cm⁻¹
ν CH : 2900 - 2960 cm⁻¹
ν C = O (éther) : 2840 cm⁻¹
ν C = O (amide) : 1640 cm⁻¹
ν NH (élongation) : 1250 cm⁻¹

### EXEMPLE 29 : N-ETHYL N-[2-(NAPHT-1-YL)ETHYL] CYCLOBUTANECARBOXAMIDE

### STADE A : N-ETHYL N-[2-(NAPHT-1-YL)ETHYL] AMINE

On préprare une solution composée de 0,03 mole de chlorhydrate de N-[2-(napht-1-yl)éthyl] amine, de 0,03 mole de chloroéthane, de 0,06 mole de carbonate de potassium et de 50 cm3 d'acétone.

On porte le mélange au reflux, sous agitation, pendant 12 heures. Après évaporation , on reprend le résidu à l'éther, puis on épuise la phase éthérée avec de l'acide chlorhydrique 1N. On alcalinise à froid la phase acide, on extrait à l'éther, et on séche. Après évaporation, on obtient la N-éthyl N-[2-(napht-1-yl)éthyl] amine.

### STADE B : N-ETHYL N-[2-(NAPHT-1-YL)ETHYL] CYCLOBUTANECARBOXAMIDE

En procédant comme dans l'exemple 1 mais en remplaçant la N-[2-(napht-1-yl)éthyl] amine par l'amine secondaire obtenue au stade précédent, on obtient la N-éthyl N-[2-(napht-1-yl)éthyl] cyclobutanecarboxamide.

### EXEMPLES 30 A 32

En procédant comme dans l'exemple 11 mais en remplaçant au stade A le 2-(napht-1-yl)acétonitrile par le 2-(5-méthoxynapht-1-yl)acétonitrile et en utilisant au stade C les halogénures d'acyle appropriés, on obtient les composés des exemples suivants :
- **EXEMPLE 30**: **: N-[2-(5-METHOXYNAPHT-1-YL) 2-(METHOXYCARBONYL)ETHYL] CYCLO BUTANECARBOXAMIDE**
- **EXEMPLE 31**: **: N-[2-(5-METHOXYNAPHT-1-YL) 2-(METHOXYCARBONYL)ETHYL] CYCLO PROPANECARBOXAMIDE**
- **EXEMPLE 32**: **: N-[2-(5-METHOXYNAPHT-1-YL) 2-(METHOXYCARBONYL)ETHYL] ACETAMIDE**

### EXEMPLE 33 : N-[2-(NAPHT-1-YL) 2-(METHOXYCARBONYL)ETHYL] N'-PROPYLUREE

A une suspension de 0,01 mole de chlorhydrate de 2-(napht-1-yl) 2-(méthoxycarbonyl)éthylamine, obtenue au stade B de l'exemple 11, dans 5 cm³ de pyridine, on ajoute goutte à goutte sous agitation magnétique 0,011 mole d'isocyanate de propyle. On agite 1 heure à une température de 80° C, puis on verse le milieu réactionnel sur de l'eau glacée. On acidifie par une solution d'acide chlorhydrique 1N. Le précipité formé est essoré, lavé à l'eau, séché, puis recristallisé dans un mélange toluène-cyclohexane.

### EXEMPLES 34 A 36

En remplaçant dans l'exemple 33 l'isocyanate de propyle par l'isocyanate correspondant, on obtient les composés des exemples suivants:
- **EXEMPLE 34**: **: N-[2-(NAPHT-1-YL) 2-(METHOXYCARBONYL)ETHYL]N'-BENZYLUREE**
- **EXEMPLE 35**: **: N-[2-(NAPHT-1-YL) 2-(METHOXYCARBONYL)ETHYL] N'-CYCLOBUTYL UREE**
- **EXEMPLE 36**: **: N-[2-(NAPHT-1-YL) 2-(METHOXYCARBONYL)ETHYL]N'-BUTYLUREE**

### EXEMPLES 37 A 39

En procédant comme dans les exemples 1 à 3 mais en remplaçant dans l'exemple 1 le 2-(napht-1-yl)acétonitrile par le 2-(napht-2-yl)acétonitrile, on obtient les composés des exemples suivants :
- **EXEMPLE 37**: **: N-[2-(NAPHT-2-YL)ETHYL] CYCLOBUTANECARBOXAMIDE**
- **EXEMPLE 38**: **: N-[2-(NAPHT-2-YL)ETHYL] CYCLOPROPANECARBOXAMIDE**
- **EXEMPLE 39**: **: N-[2-(NAPHT-2-YL)ETHYL] TRIFLUOROMETHYLCARBOXAMIDE**

### EXEMPLE 40 : N-[2-(NAPHT-2-YL) 2-(METHOXYCARBONYL)ETHYL] CYCLOBUTANECARBOXAMIDE

En procédant comme dans l'exemple 11 mais en remplaçant au stade A le 2-(napht-1-yl)acétonitrile par le 2-(napht-2-yl)acétonitrile, on obtient le N-[2-(napht-2-yl) 2-(méthoxycarbonyl)éthyl] cyclobutyl carboxamide.

### EXEMPLE 41 : N-[2-(7-HYDROXYNAPHT-1-YL)ETHYL] CYCLOBUTANECARBOXAMIDE

En procédant comme dans l'exemple 24, mais en remplaçant le chlorure de l'acide cyclopropanecarboxylique par le chlorure de l'acide cyclobutanecarboxylique, on obtient le N-[2-(7-hydroxynapht-1-yl)éthyl] cyclobutanecarboxamide.

### EXEMPLE 42 : N-[2-[7-(CYCLOHEXENE-3-YL)OXYNAPHT-1-YL]ETHYL] CYCLOBUTANECARBOXAMIDE

En procédant comme dans l'exemple 25, mais en remplaçant le composé obtenu à l'exemple 24 par le composé obtenu à l'exemple 41, on obtient le N-{2-[7-cyclohexène-3-yl)oxynapht-1-yl]éthyl} cyclobutylanecarboxamide.
Point de fusion : 104-106° C

| Microanalyse : | | | |
|---|---|---|---|
| % | C | N | H |
| théorique | 79,04 | 7,78 | 4,01 |
| calculé | 78,60 | 7,72 | 3,88 |

### EXEMPLE 43 : N-[2-(7-CYCLOHEXYLOXYNAPHT-1-YL)ETHYL] CYCLOBUTANECARBOXAMIDE

En procédant comme dans l'exemple 28, mais en remplaçant le composé obtenu à l'exemple 25 par le composé obtenu à l'exemple 42, on obtient le N-[2-(7-cyclohexyloxynapht-1-yl)éthyl] cyclobutanecarboxamide.
Point de fusion : 116-118° C

### EXEMPLE 44 : N-[2-(7-HYDROXYNAPHT-1-YL)ETHYL] ACETAMIDE

En procédant comme dans l'exemple 24, mais en remplaçant le chlorure de l'acide cyclopropanecarboxylique par l'anhydride acétique, on obtient le N-[2-(7-hydroxynapht-1-yl)éthyl] acétamide.
Point de fusion : 125-126° C

### ETUDE PHARMACOLOGIQUE

### EXEMPLE A : ETUDE DE LA TOXICITE AIGUE

La toxicité aiguë a été appréciée après administration orale à des lots de 8 souris (26±2 grammes). Les animaux ont été observés à intervalles réguliers au cours de la première journée et quotidiennement pendant les deux semaines suivant le traitement. La DL 50, entraînant la mort de 50 %, des animaux, a été évaluée.

La DL 50 des produits testés est supérieure à 1000 mg.kg⁻¹ pour la plupart des composés étudiés ce qui indique la faible toxicité des composés de l'invention.

### EXEMPLE B : ETUDE DE LIAISON AUX RECEPTEURS DE LA MELATONINE

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology vol (1), pp 1-4 (1989)).

### PROTOCOLE

1) Les membranes de pars Tuberalis de mouton sont préparées et utilisées comme tissu cible dans des expériences de saturation pour déterminer ses capacités et affinités de liaisons pour la ¹²⁵I - iodomélatonine.
2) Les membranes de Pars tuberalis de mouton sont utilisées comme tissu cible, avec les différents composés à tester, dans des expériences de liaison compétitive par rapport à la mélatonine.

Chaque expérience est réalisée en triplicate et une gamme de concentrations différentes est testée pour chaque compose.

Les résultats permettent de déterminer, après traitement statistique, les affinités de liaison du composé testé.

### RESULTATS

Il apparaît que les composés de l'invention possèdent une puissante affinité pour les récepteurs de la mélatonine puisqu'ils se fixent avec une constante de dissociation de l'ordre de 10⁻¹¹ M.

### EXEMPLE C : ETUDE DE L'ACTIVITE ANTAGONISTE DE LA MELATONINE DES COMPOSES DE L'INVENTION

L'activité antagoniste des composés de l'invention peut être mesurée sur les cellules de la Pars Tuberalis de mouton préalablement stimulées par la forskoline, cellules dans lesquelles la mélatonine inhibe de façon spécifique la synthèse d'AMP cyclique. (J. Mol. Endocr. 1989; 3; R5-R8)

### PROTOCOLE

Les cellules de Pars Tuberalis de mouton en culture sont incubées en présence de forskoline (10⁻⁶ M) et du composé à tester, seul ou en combinaison avec la mélatonine (10⁻⁹ M).

Le dosage de l'AMP cyclique est réalisé par un test radio immunologique.

Chaque expérience est réalisée en triplicate et plusieurs concentrations sont testées pour chaque composé afin d'établir la courbe dose-réponse et de déterminer la concentration inhibant de 50 % l'action de la mélatonine (ou IC₅₀).

### RESULTATS

La demanderesse a découvert que les composés de l'invention possédaient une activité antagoniste très puissante et largement supérieure aux valeurs connues dans la littérature. Par contre, dans ce test, les composés de la demande EP 447285 se révélent posséder une intense activité agoniste des récepteurs de la mélatonine.

### EXEMPLE D : ETUDE IN VIVO DE L'ACTIVITE ANTAGONISTE DE LA MELATONINE DES COMPOSES DE L'INVENTION

Chez le hamster doré mâle adulte maintenu en photopériode longue (cycle lumière/obscurité : 14 H/10 H; dans ces conditions, son axe sexuel est actif), si l'on injecte quotidiennement, en fin de période d'éclairement, de la mélatonine, cette hormone est capable d'induire, après 8 semaines, un arrêt total de l'axe sexuel (atrophie des gonades).

En agissant sur la température ambiante, il a été possible de développer plus avant ce test et maintenant cette atrophie des gonades peut être obtenue en 4 semaines seulement.

Dans ces conditions, un composé capable d'empêcher l'effet de la mélatonine est considéré comme un antagoniste des récepteurs de la mélatonine

### PROTOCOLE

Des groupes de 12 hamsters dorés adultes, sont placés en situation de photopériode longue (cycle lumière/obscurité : 14 heures /10 heures, nuit de 18 heures à 4 heures) à une température de 7° C.

En fin de chaque période d'éclairement (entre 16 et 17 heures) on injecte à chaque animal
- soit du solvant (groupe témoin solvant)
- soit la mélatonine seule (10 microgrammes/jour, groupe témoin mélatonine)
- soit le composé à tester seul (groupe témoin composé à tester)
- soit le composé à tester et la mélatonine.

Les animaux sont sacrifiés après 4 semaines et l'atrophie des gonades est mesurée.

### RESULTATS

Il apparait que les composés de l'invention possèdent in vivo un fort pouvoir antagoniste de la mélatonine. Par opposition, les composés de la demande EP 447285 se révélent posséder, dans ce test, une importante activité agoniste des récepteurs de la mélatonine.

### EXEMPLE E : ETUDE DE L'ACTIVITE ANTAGONISTE DE LA MELATONINE DES COMPOSES SUR L'AGREGATION PIGMENTAIRE

La mélatonine est connue pour participer à la régulation de la couleur de la peau des amphibiens tels que le xénope (Xenopus laevis). Elle provoque la condensation des granules contenant de la mélanine dans les mélanophores du derme (Sudgen D. European Journal of Pharmacology, vol. 213, pp : 405-408 (1992)).

Les composés de l'invention ont été testés sur leur capacité à inhiber l'effet de la mélatonine sur des mélanophores de xénope.

### PROTOCOLE

(selon la méthode décrite dans Sudgen D. Br. J. Pharmacol, vol. 104, pp 922-927, (1991)).

La crête neurale d'embryons de xénope est disséquée et les cellules sont isolées. Les mélanophores qui apparaissent après 2-3 jours de culture sont rapidement visibles parmi les autres cellules issues de la crète neural. Les mélanophores sont cultivées dans du milieu de Leibovitz L-15 (Gibco) et dilués (1/1) avec de l'eau déionisée contenant 10 % de sérum de veau foetal, 200 UI/cm³ de penicilline, 200 µg/cm³ de streptomycine, 2,5 µg/cm³ d'amphotéricine B.
Les expérimentations sur les mélanophores sont réalisées entre le 7ème et le 12ème jour de culture.
La réponse à la mélatonine des mélanophores isolées est quantifiée par la mesure de la surface pigmentée grâce à un analyseur d'image assisté par ordinateur. Les composés à tester sont ajoutés au milieu de culture 10 à 15 min avant la mesure.

### RESULTATS

Il apparaît que les composés de l'invention antagonisent de façon importante l'activité de la mélatonine.
A titre d'exemple, le composé de l'exemple 1 antagonise de façon significative l'action de la mélatonine (10⁻⁸ M) à une concentration de 10⁻⁷ M.

### EXEMPLE F : COMPOSITION PHARMACEUTIQUE : COMPRIMES

Comprimés dosés à 5 mg de N-[2-(napht-1-yl)éthyl] cyclobutanecarboxamide.

Formule de préparation pour 1000 comprimés :

| | |
|---|---|
| N-[2-(napht-1-yl)éthyl] cyclobutanecarboxamide | 5 g |
| Amidon de blé | 1,5 g |
| Amidon de maïs | 1,5 g |
| Lactose | 6,5 g |
| Stéarate de magnésium | 0,2 g |
| Silice | 0,1 g |
| Hydroxypropylcellulose | 0,2 g |

## Revendications

1. Composés de formule générale (I) : 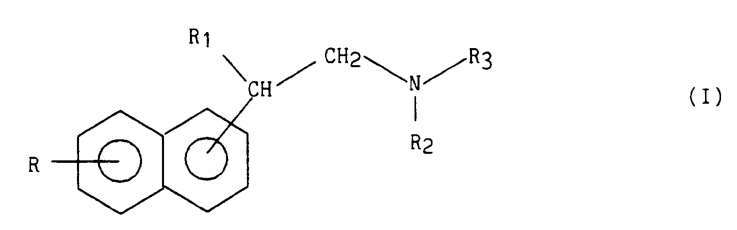 dans laquelle :
R représente un atome d'hydrogène ou un groupement -O-R₄ dans lequel R₄ signifie un atome d'hydrogène ou un groupement, substitué ou non substitué, choisi parmi alkyle, cycloalkyle, cycloalkylalkyle, phényle, phénylalkyle, et diphénylalkyle,
R₁ représente un atome d'hydrogène ou un groupement -CO-O-R₅ dans lequel R₅ signifie un atome d'hydrogène ou un groupement alkyle substitué ou non substitué,
R₂ représente un atome d'hydrogène ou un groupement - R'₂ avec R'₂ représentant un radical alkyle ou alkyle substitué,
R₃ représente :
- un groupement 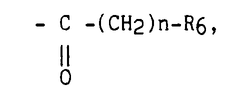 dans lequel n représente 0 ou un
nombre entier de 1 à 3, et R₆ représente un atome d'hydrogène, ou un groupement alkyle, alkyle substitué, alcène, alcène substitué, cycloalkyle, cycloalkyle substitué, ou un groupement hétérocyclique, substitué ou non substitué, choisi parmi : pyrrolidine, pipéridine, pipérazine, homopipéridine, homopipérazine, morpholine, et thiomorpholine ;
- un groupement 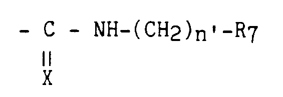 dans lequel X représente un atome d'oxygène ou de soufre, n' représente 0 ou un nombre entier de 1 à 3, et R₇ représente un groupement alkyle, alkyle substitué, cycloalkyle, cycloalkyle substitué, phényle, ou phényle substitué,
étant entendu que si :
- R représente un groupement alcoxy,
- R représente un atome d'hydrogène et R₃ représente un groupement -CO-R₈, dans lequel R₈ représente un atome d'hydrogène, un groupement méthyle, ou un groupement méthyle ou propyle substitué par un halogène,
- ou si R₃ représente un groupement 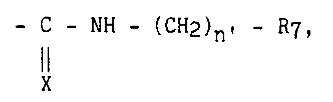 dans lequel X, n', et R₇ sont tels que définis précédemment,
alors R₁ ne peut pas être un atome d'hydrogène,
leurs isomères optiques, et leurs sels d'addition à une base pharmaceutiquement acceptable,
étant entendu que, sauf précisions contraires,
- le terme "substitué" signifie que les groupements auxquels il se rapporte peuvent être substitués par un ou plusieurs radicaux choisis parmi halogène, alkyle (C₁-C₄), alcoxy (C₁-C₄), phényle, et phénylalkyle, les noyaux phényles pouvant eux-mêmes être substitués par un ou plusieurs radicaux halogène, alkyle (C₁-C₄), alcoxy (C₁-C₄), hydroxyle, ou trifluorométhyle,
- le terme "alkyle" signifie un groupement comportant de 1 à 6 atomes de carbone en chaîne droite ou ramifiée,
- le terme "alcène" signifie un groupement comportant de 2 à 6 atomes de carbone en chaine droite ou ramifiée,
- le terme "cycloalkyle" signifie un groupement contenant de 3 à 10 atomes de carbone, mono- ou bi-cyclique, saturé ou insaturé.

2. Composés de formule (I) selon la revendication 1 dans lesquels R représente un atome d'hydrogène, leurs isomères optiques, et leurs sels d'addition à une base pharmaceutiquement acceptable.

3. Composés de formule (I) selon la revendication 1 dans lesquels R₁ représente un groupement -CO-O-R₅ dans lequel R₅ signifie un groupement alkyle, leurs isomères optiques, et leurs sels d'addition à une base pharmaceutiquement acceptable.

4. Composés de formule (I) selon la revendication 1 dans lesquels R₃ représente un groupement : 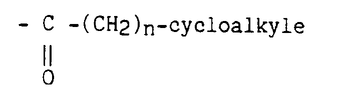 substitué ou non substitué avec les termes "n" et "cycloalkyle" tels que définis dans la formule (I) selon la revendication 1, leurs isomères optiques, et leurs sels d'addition à une base pharmaceutiquement acceptable.

5. Composés de formule (I) selon la revendication 1 dans lesquels :
- R représente un atome d'hydrogène,
- R₁ représente un groupement -CO-O-R₅ dans lequel R₅ signifie un groupement alkyle,
- R₃ représente un groupement 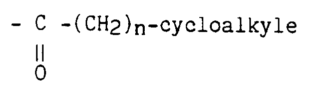 avec les termes "n" et "cycloalkyle" tels que définis dans la formule (I) selon la revendication 1,
et leurs isomères optiques.

6. Composé de formule (I) selon la revendication 1 qui est le N-[2-(napht-1-yl)éthyl] cyclobutanecarboxamide.

7. Composé de formule (I) selon la revendication 1 qui est le N-[2-(napht-1-yl)éthyl] cyclopropanecarboxamide.

8. Composé de formule (I) selon la revendication 1 qui est le N-[2-(napht-1-yl)éthyl] trifluoroacétamide.

9. Composé de formule (I) selon la revendication 1 qui est le N-[2-(napht-1-yl) 2-(méthoxycarbonyl)éthyl] cyclobutanecarboxamide et ses isomères optiques.

10. Composé de formule (I) selon la revendication 1 qui est le N-[2-(napht-1-yl) 2-(méthoxycarbonyl)éthyl] cyclopropanecarboxamide et ses isomères optiques.

11. Composé de formule (I) selon la revendication 1 qui est le N-{2-[7-(cyclohexène-3-yl)oxynapht-1-yl]éthyl} cyclopropanecarboxamide.

12. Composé de formule (I) selon la revendication 1 qui est le N-[2-(7-cyclohexyloxynapht-1-yl]éthyl} cyclopropanecarboxamide.

13. Composé de formule (I) selon la revendication 1 qui est le N-[2-(7-hydroxynapht-1-yl)éthyl]acétamide.

14. Composé de formule (I) selon la revendication 1 qui est le N-[2-(7-benzyloxynapht-1-yl)éthyl] cyclopropanecarboxamide.

15. Procédé de préparation des composés de formule (I) selon la revendication 1 caractérisé en ce que : on fait réagir un composé de formule (II) : 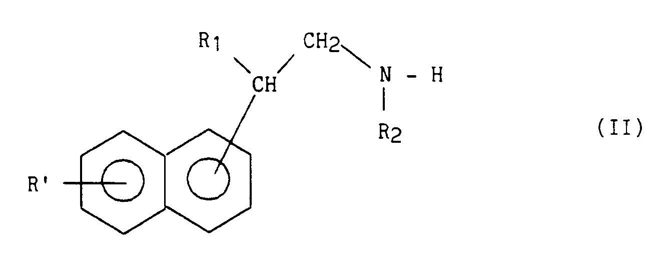 dans laquelle R₁ et R₂ sont tels que définis dans la formule (I) selon la revendication 1 et R' représente un atome d'hydrogène, un groupement hydroxyle, ou un groupement alcoxy,
- soit
. avec un halogénure d'acide de formule (III) 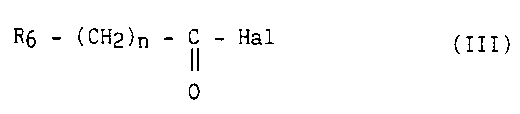 dans laquelle R₆ et n sont tels que définis dans la formule (I) selon la revendication 1 et Hal représente un atome d'halogène,
. ou avec un composé de formule (III/a), lors d'une hydrogénation catalytique, 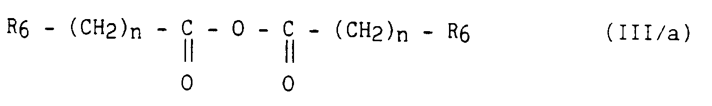
dans laquelle R₆ et n sont tels que définis précédemment, pour obtenir un composé de formule (I/a) 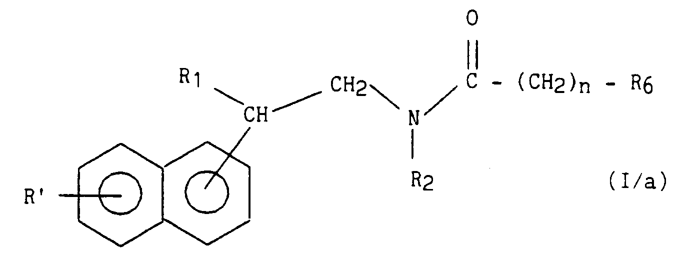 dans laquelle R', R₁, R₂, R₆, et n sont tels que définis précédemment, cas particulier des composés de formule (I) selon la revendication 1 dans lesquels R représente un groupement R' et R₃ représente un groupement 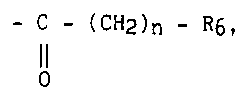
- soit avec un isocyanate ou un isothiocyanate de formule (IV) :
X = C = N -(CH₂)_{n'}- R₇ (IV)
dans laquelle X, n', et R₇ sont tels que définis dans la formule (I) selon la revendication 1 pour obtenir un composé de formule (I/c) : 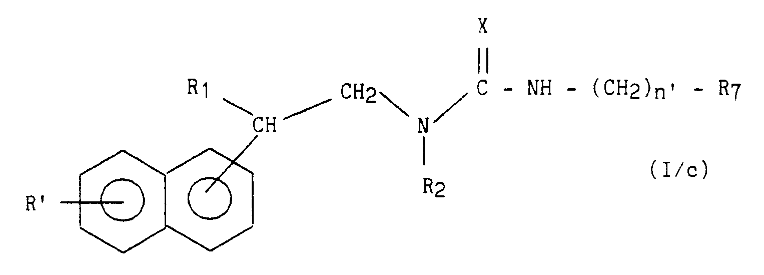
dans laquelle R', R₁, R₂, R₇ , X, et n' sont tels que définis précédemment, cas particulier des composés de formule (I) dans lesquels R représente un groupement R' et R₃ représente un groupement 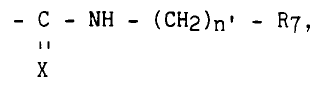 composés de formules (I/a), (I/b), et (I/c) qui peuvent être également, lorsque R' représente un groupement hydroxyle, mis en réaction avec un composé de formule (V)
R'' - Hal' (V)
dans laquelle R'' représente un groupement, non substitué ou substitué (le terme substitué étant tel que défini dans la revendication 1), choisi parmi cycloalkyle, cycloakylalkyle, phényle, phénylalkyle, et diphénylalkyle, et Hal' représente un atome d'halogène afin d'obtenir un composé de formule (I/d) 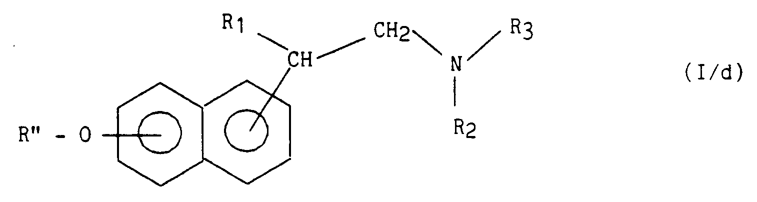 dans laquelle R₁, R₂, R₃, et R'' sont tels que définis précédemment, cas particulier des composés de formule (I) selon la revendication 1 dans lesquels R représente un groupement -O- R'', les composés de formules (I/a), (I/b), (I/c), et (I/d) formant l'ensemble des composés de formule (I) selon la revendication 1,
les composés de formule (I) selon la revendication 1 pouvant être :
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels isomères optiques,
- ou salifiés, dans le cas où R₁ représente un groupement carboxyle ou dans le cas où R représente un groupement hydroxyle, par une base pharmaceutiquement acceptable.

16. Procédé d'obtention des composés de formule (I/e) 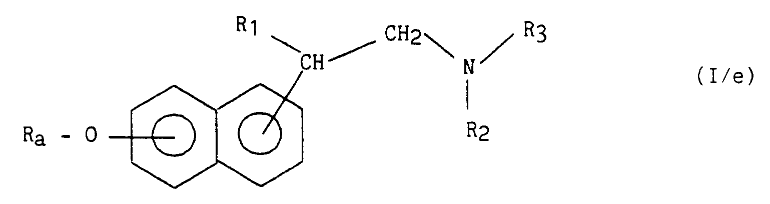 dans laquelle R₁, R₂, et R₃ sont tels que définis dans la formule (I) et Rₐ représente un groupement, saturé, cycloalkyle ou cycloalkylalkyle,
cas particulier des composés de formule (I) selon la revendication 1 dans lequel R représente un groupement -O-Rₐ,
caractérisé en ce que l'on soumet un composé de formule (I/f) 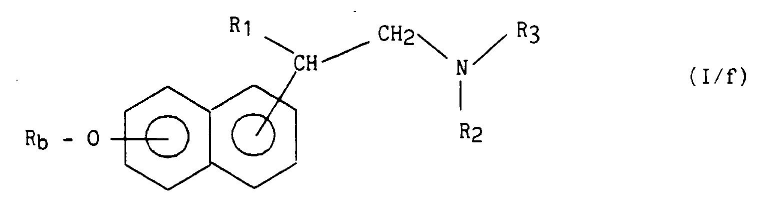 dans laquelle R₁, R₂, et R₃ sont tels que définis précédemment et R_{b} représente un groupement, insaturé, cycloalcényle ou cycloalcènylalkyle,
cas particulier des composés de formule (I) selon la revendication 1 dans lesquels R représente un groupement -O-R_{b},
à une hydrogénation catalytique pour obtenir le composé de formule (I/a) où Rₐ correspond à la forme saturée du groupement R_{b},
les composés de formule (I/e) pouvant être :
- purifiés suivant une ou plusieurs méthodes de purification choisies parmi la cristallisation, la chromatographie sur gel de silice, l'extraction, la filtration , et le passage sur charbon ou résine,
- séparés, le cas échéant, sous forme pure ou sous forme de mélange, en leurs éventuels isomères optiques,
- ou salifiés, dans le cas où R₁ représente un groupement carboxyle, par une base pharmaceutiquement acceptable.

17. Composition pharmaceutique contenant comme principe actif au moins un des composés de formule I selon la revendication 1, en combinaison avec un ou plusieurs excipients ou véhicules inertes non toxiques, pharmaceutiquement acceptables.

18. Composition pharmaceutique selon la revendication 17 utile dans le traitement des troubles liés à une activité anormale de la mélatonine dans l'organisme, ainsi que dans le traitement des troubles du système nerveux central, du système immunitaire, et du système endocrinien.

## Claims

1. Compounds of the general formula (I) : 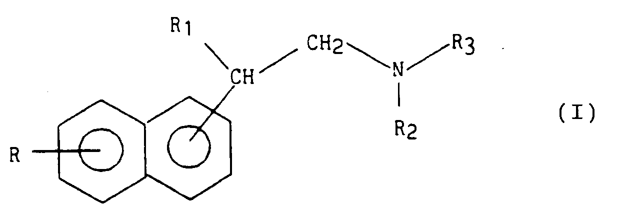 wherein :
R represents a hydrogen atom or an -O-R₄ group wherein R₄ represents a hydrogen atom or a substituted or unsubstituted group selected from alkyl, cycloalkyl, cycloalkylalkyl, phenyl, phenylalkyl and diphenylalkyl,
R₁ represents a hydrogen atom or a -CO-O-R₅ group wherein R₅ represents a hydrogen atom or a substituted or unsubstituted alkyl group,
R₂ represents a hydrogen atom or a - R'₂ group, R'₂ representing an alkyl or substituted alkyl radical,
R₃ represents : 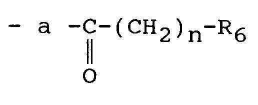 group wherein n represents O or an integer of from 1 to 3, and R₆ represents a hydrogen atom, an alkyl, substituted alkyl, alkenyl, substituted alkenyl, cycloalkyl or substituted cycloalkyl group, or a substituted or unsubstituted heterocyclic group selected from : pyrrolidine, piperidine, piperazine, homopiperidine, homopiperazine, morpholine and thiomorpholine;
- a 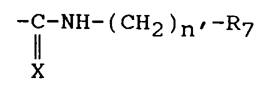 group wherein X represents an oxygen or sulphur atom, n' represents O or an integer of from 1 to 3 and R₇ represents an alkyl, substituted alkyl, cycloalkyl, substituted cycloalkyl, phenyl or substituted phenyl group,
with the proviso that if:
- R represents an alkoxy group,
- R represents a hydrogen atom and R₃ represents a -CO-R₈ group wherein R₈ represents a hydrogen atom, a methyl group or a methyl or propyl group substituted by a halogen,
- or if R₃ represents a 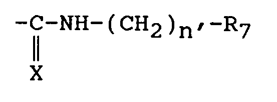 group wherein X, n' and R₇ are as defined above,
then R₁ cannot represent a hydrogen atom,
their optical isomers, and their addition salts with a pharmaceutically acceptable base,
it being understood that, unless specified to the contrary,
- the term "substituted" denotes that the groups to which it relates may be substituted by one or more radicals selected from halogen, (C₁-C₄)alkyl, (C₁-C₄)alkoxy, phenyl and phenylalkyl, it being possible for the phenyl nuclei themselves to be substituted by one or more halogen, (C₁-C₄)alkyl, (C₁-C₄)alkoxy, hydroxy or trifluoromethyl radicals,
- the term "alkyl" denotes a group having from 1 to 6 carbon atoms in straight or branched chain,
- the term "alkenyl" denotes a group having from 2 to 6 carbon atoms in straight or branched chain,
- the term "cycloalkyl" denotes a saturated or unsaturated, mono- or bi-cyclic group containing from 3 to 10 carbon atoms.

2. Compounds of formula (I) according to claim 1, wherein R represents a hydrogen atom, their optical isomers, and their addition salts with a pharmaceutically acceptable base.

3. Compounds of formula (I) according to claim 1, wherein R₁ represents a -CO-O-R₅ group in which R₅ represents an alkyl group, their optical isomers, and their addition salts with a pharmaceutically acceptable base.

4. Compounds of formula (I) according to claim 1, wherein R₃ represents a substituted or unsubstituted 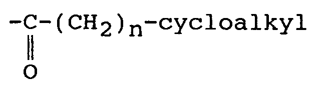 group, the terms "n" and "cycloalkyl" being as defined for formula (I) according to claim 1, their optical isomers, and their addition salts with a pharmaceutically acceptable base.

5. Compounds of formula (I) according to claim 1 wherein :
- R represents a hydrogen atom,
- R₁ represents a -CO-O-R₅ group wherein R₅ represents an alkyl group,
- R₃ represents a 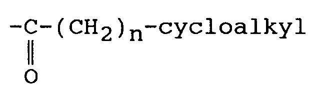 group, the terms "n" and "cycloalkyl" being as defined for formula (I) according to claim 1,
and their optical isomers.

6. Compound of formula (I) according to claim 1, which is N-[2-(naphth-1-yl)ethyl]cyclobutanecarboxamide.

7. Compound of formula (I) according to claim 1, which is N-[2-(naphth-1-yl)ethyl]cyclopropanecarboxamide.

8. Compound of formula (I) according to claim 1, which is N-[2-(naphth-1-yl)ethyl]trifluoroacetamide.

9. Compound of formula (I) according to claim 1, which is N-[2-(naphth-1-yl)-2-(methoxycarbonyl)ethyl]cyclobutanecarboxamide and its optical isomers.

10. Compound of formula (I) according to claim 1, which is N-[2-(naphth-1-yl)-2-(methoxycarbonyl)ethyl]cyclopropanecarboxamide and its optical isomers.

11. Compound of formula (I) according to claim 1, which is N-{2-[7-(cyclohexen-3-yl)oxynaphth-1-yl]ethyl}cyclopropanecarboxamide.

12. Compound of formula (I) according to claim 1, which is N-[2-(7-cyclohexyloxynaphth-1-yl)ethyl]cyclopropanecarboxamide.

13. Compound of formula (I) according to claim 1, which is N-[2-(7-hydroxynaphth-1-yl)ethyl]acetamide.

14. Compound of formula (I) according to claim 1, which is N-[2-(7-benzyloxynaphth-1-yl)ethyl]cyclopropanecarboxamide.

15. Process for the preparation of compounds of formula (I) according to claim 1, characterised in that : a compound of formula (II) : 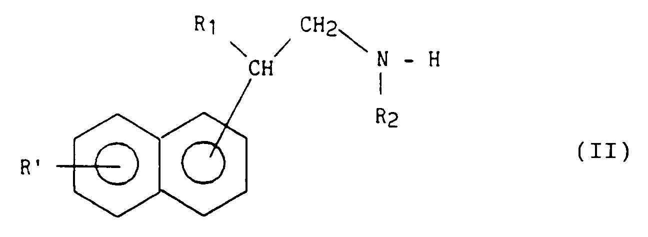 wherein R₁ and R₂ are as defined for formula (I) according to claim 1 and R' represents a hydrogen atom, a hydroxy group or an alkoxy group, is reacted
- either
. with an acid halide of formula (III) 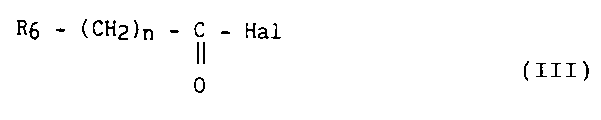 wherein R₆ and n are as defined for formula (I) according to claim 1 and Hal represents a halogen atom,
. or with a compound of formula (III/a), with catalytic hydrogenation, 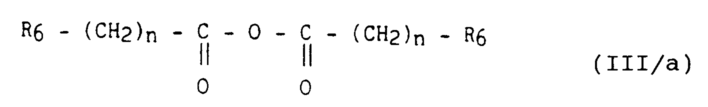
wherein R₆ and n are as defined above, to obtain a compound of formula (I/a) 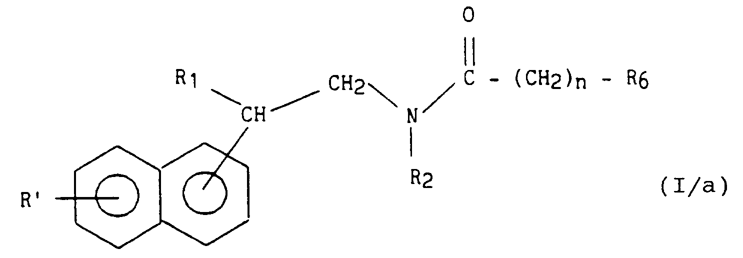 wherein R', R₁, R₂, R₆ and n are as defined above, a particular case of compounds of formula (I) according to claim 1 wherein R represents a group R' and R₃ represents a 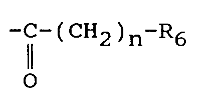 group
- or with an isocyanate or an isothiocyanate of formula (IV) :
X=C=N-(CH₂)_{n'}-R₇ (IV)
wherein X, n' and R₇ are as defined for formula (I) according to claim 1, to obtain a compound of formula (I/c) : 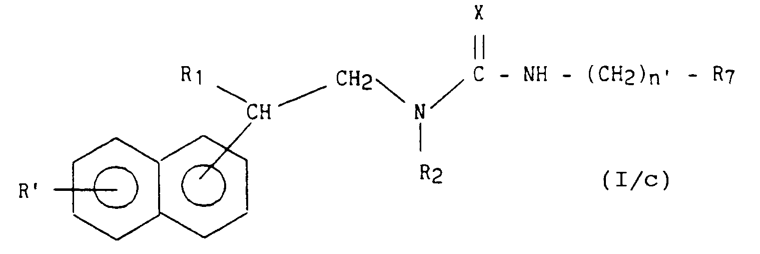 wherein R', R₁, R₂, R₇, X and n' are as defined above, a particular case of compounds of formula (I) wherein R represents a group R' and R₃ represents a 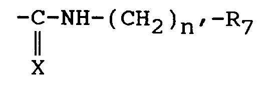 group, which compounds of formulae (I/a), (I/b) and (I/c) may also, when R' represents a hydroxy group, be reacted with a compound of formula (V)
R''-Hal' (V)
wherein R'' represents an unsubstituted or substituted group (the term substituted being as defined in claim 1) selected from cycloalkyl, cycloalkylalkyl, phenyl, phenylalkyl and diphenylalkyl, and Hal' represents a halogen atom, to obtain a compound of formula (I/d) 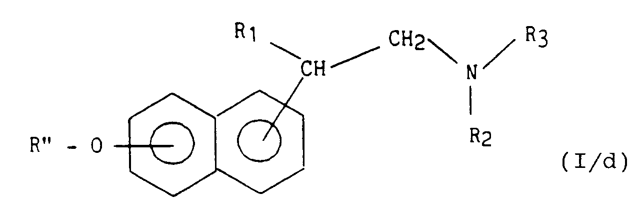 wherein R₁, R₂, R₃ and R'' are as defined above, a particular case of compounds of formula (I) according to claim 1 wherein R represents an -O-R'' group,
the compounds of formulae (I/a), (I/b), (I/c) and (I/d) forming the totality of the compounds of formula (I) according to claim 1,
it being possible for the compounds of formula (I) according to claim 1 to be :
- purified according to one or more methods of purification selected from crystallisation, chromatography on silica gel, extraction, filtration and passage over carbon or resin,
- separated, where appropriate, in pure form or in the form of a mixture, into their possible optical isomers,
- or converted into salts, when R₁ represents a carboxy group or when R represents a hydroxy group, by a pharmaceutically acceptable base.

16. Process for obtaining compounds of formula (I/e) 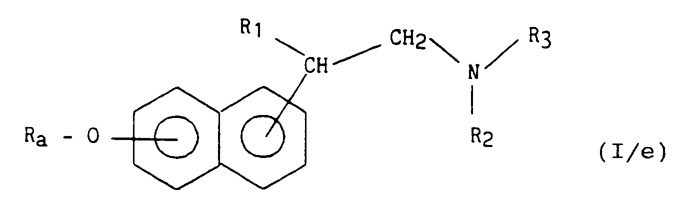 wherein R₁, R₂ and R₃ are as defined for formula (I) and Rₐ represents a saturated cycloalkyl or cycloalkylalkyl group,
a particular case of compounds of formula (I) according to claim 1 wherein R represents an -O-Rₐ group,
characterised in that a compound of formula (I/f) 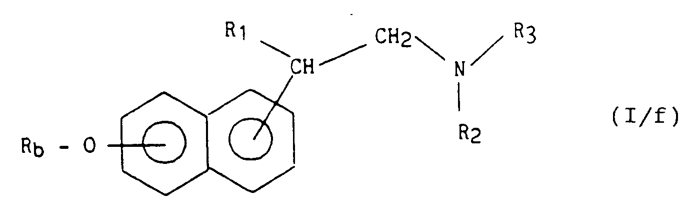 wherein R₁, R₂ and R₃ are as defined above and R_{b} represents an unsaturated cycloalkenyl or cycloalkenylalkyl group,
a particular case of compounds of formula (I) according to claim 1 wherein R represents an -O-R_{b} group,
is subjected to catalytic hydrogenation to obtain the compound of formula (I/a) wherein Rₐ corresponds to the saturated form of the group R_{b},
it being possible for the compounds of formula (I/e) to be :
- purified according to one or more methods of purification selected from crystallisation, chromatography on silica gel, extraction, filtration and passage over carbon or resin,
- separated, where appropriate, in pure form or in the form of a mixture, into their possible optical isomers,
- or converted into salts, when R₁ represents a carboxy group, by a pharmaceutically acceptable base.

17. Pharmaceutical composition comprising as active ingredient at least one of the compounds of formula I according to claim 1, in combination with one or more pharmaceutically acceptable, inert, non-toxic excipients or carriers.

18. Pharmaceutical composition according to claim 17 for use in the treatment of disorders associated with an abnormal melatonin activity in the organism, as well as in the treatment of disorders of the central nervous system, the immune system or the endocrine system.

## Patentansprüche

1. Verbindungen der allgemeinen Formel (I): 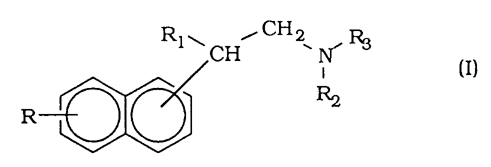 in der:
R ein Wasserstoffatom oder eine Gruppe -O-R₄, in der R₄ ein Wasserstoffatom oder eine gegebenenfalls substituierte Gruppe ausgewählt aus Alkyl-, Cycloalkyl-, Cycloalkylalkyl-, Phenyl-, Phenylalkyl- und Diphenylalkylgruppen darstellt,
R₁ ein Wasserstoffatom oder eine Gruppe -CO-O-R₅, in der R₅ ein Wasserstoffatom oder eine gegebenenfalls substituierte Alkylgruppe darstellt,
R₂ ein Wasserstoffatom oder eine Gruppe -R'₂, worin R'₂ eine Alkylgruppe oder eine substituierte Alkylgruppe darstellt,
R₃:
- eine Gruppe 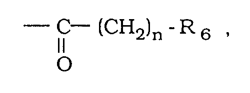 in der n 0 oder eine ganze Zahl mit einem Wert von 1 bis 3 und R₆ ein Wasserstoffatom, eine Alkylgruppe, eine substituierte Alkylgruppe, eine Alkengruppe, eine substituierte Alkengruppe, eine Cycloalkylgruppe, eine substituierte Cycloalkylgruppe oder eine gegebenenfalls substituierte heterocyclische Gruppe ausgewählt aus Pyrrolidin- Piperidin-, Piperazin-, Homopiperidin-, Homopiperazin-, Morpholin- und Thiomorpholingruppen;
- eine Gruppe 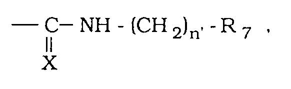 in der X ein Sauerstoffatom oder ein Schwefelatom, n' 0 oder eine ganze Zahl mit einem Wert von 1 bis 3 und R₇ eine Alkylgruppe, eine substituierte Alkylgruppe, eine Cycloalkylgruppe, eine substituierte Cycloalkylgruppe, eine Phenylgruppe oder eine substituierte Phenylgruppe darstellt,
mit der Maßgabe bedeuten, daß, wenn:
- R eine Alkoxygruppe,
- R ein Wasserstoffatom und R₃ eine Gruppe -CO-R₈, worin R₈ ein Wasserstoffatom, eine Methylgruppe oder eine durch ein Halogen substituierte Methyl- oder Propylgruppe darstellt,
- oder, wenn R₃ eine Gruppe 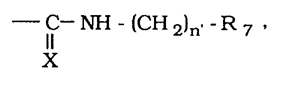 in der X, n' und R₇ die oben angegebenen Bedeutungen besitzen, darstellen,
R₁ kein Wasserstoffatom bedeutet,
deren optische Isomeren und deren Additionssalze mit einer pharmazeutisch annehmbaren Base,
mit der Maßgabe, daß, wenn nichts anderes angegeben ist,
- der Begriff"substituiert"bedeutet, daß die Gruppen, aufwelche er sich bezieht, durch eine oder mehrere Gruppen ausgewählt aus Halogenatomen, (C₁-C₄)-Alkylgruppen, (C₁-C₄)-Alkoxygruppen, Phenylgruppen und Phenylalkylgruppen substituiert sind, wobei die Phenylkerne ihrerseits durch eine oder mehrere Halogenatome, (C₁-C₄)-Alkylgruppen, (C₁-C₄)-Alkoxygruppen, Hydroxylgruppen oder Trifluormethylgruppen substituiert sein können,
- der Begriff "Alkyl" für eine Gruppe mit 1 bis 6 Kohlenstoffatomen in gerader oder verzweigter Kette steht,
- der Begriff "Alken" für eine Gruppe mit 2 bis 6 Kohlenstoffatomen in gerader oder verzweigter Kette steht,
- der Begriff "Cycloalkyl" für eine mono- oder bicyclische, gesättigte oder ungesättigte Gruppe mit 3 bis 10 Kohlenstoffatomen steht.

2. Verbindungen der Formel (I) nach Anspruch 1, worin R ein Wasserstoffatom bedeutet, deren optische Isomeren und deren Additionssalze mit einer pharmazeutisch annehmbaren Base.

3. Verbindungen der Formel (I) nach Anspruch 1, worin R₁ eine Gruppe -CO-O-R₅, in der R₅ eine Alkylgruppe darstellt, bedeutet, deren optische Isomeren und deren Additionssalze mit einer pharmazeutisch annehmbaren Base.

4. Verbindungen der Formel (I) nach Anspruch 1, worin R₃ eine gegebenenfalls substituierte Gruppe: 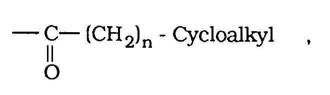 worin die Begriff "n" und "Cycloalkyl" die in Anspruch 1 bezüglich der Formel (I) angegebenen Bedeutungen besitzen, darstellt, deren optische Isomeren und deren Additionssalze mit einer pharmazeutisch annehmbaren Base.

5. Verbindungen der Formel (I) nach Anspruch 1, worin
- R ein Wasserstoffatom,
- R₁ eine Gruppe -CO-O-R₅, in der R₅ eine Alkylgruppe darstellt und
- R₃ eine Gruppe 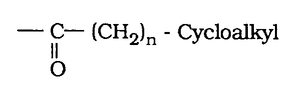 bedeuten, worin die Begriffe "n" und "Cycloalkyl" die bezüglich der Formel (I) in Anspruch 1 angegebenen Bedeutungen besitzen,
und deren optische Isomeren.

6. Verbindung der Formel (I) nach Anspruch 1, nämlich N-[2-(Naphth-1-yl)-ethyl]-cyclobutancarboxamid.

7. Verbindung der Formel (I) nach Anspruch 1, nämlich N-[2-(Naphth-1-yl)-ethyl]-cyclopropancarboxamid.

8. Verbindung der Formel (I) nach Anspruch 1, nämlich N-[2-(Naphth-1-yl)-ethyl]-trifluoracetamid.

9. Verbindung der Formel (I) nach Anspruch 1, nämlich N-[2-(Naphth-1-yl)-2-(methoxycarbonyl)-ethyl]-cyclobutancarboxamid und dessen optische Isomeren.

10. Verbindung der Formel (I) nach Anspruch 1, nämlich N-[2-(Naphth-1-yl)-2-(methoxycarbonyl)-ethyl]-cyclopropancarboxamid und dessen optische Isomeren.

11. Verbindung der Formel (I) nach Anspruch 1, nämlich N-{2-[7-(Cyclohexen-3-yl)-oxynaphth-1-yl]-ethyl}-cyclopropancarboxamid.

12. Verbindung der Formel (I) nach Anspruch 1, nämlich N-[2-(7-Cyclohexyloxynapth-1-yl)-ethyl]-cyclopropancarboxamid.

13. Verbindung der Formel (I) nach Anspruch 1, nämlich N-[2-(7-Hydroxynaphth-1-yl)-ethyl]-acetamid.

14. Verbindung der Formel (I) nach Anspruch 1, nämlich N-[2-(7-Benzyloxynaphth-1-yl)-ethyl]-cyclopropancarboxamid.

15. Verfahren zur Herstellung der Verbindungen der Formel (I) nach Anspruch 1, **dadurch gekennzeichnet**, daß man:
eine Verbindung der Formel (II): 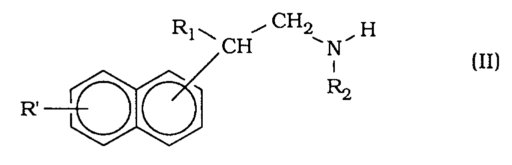 in der R₁ und R₂ die bezüglich der Formel (I) in Anspruch 1 angegebenen Bedeutungen besitzen und R' ein Wasserstoffatom, eine Hydroxylgruppe oder eine Alkoxygruppe darstellt,
- entweder mit einem Säurehalogenid der Formel (III): 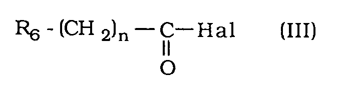 in der R₆ und n die bezüglich der Formel (I) in Anspruch 1 angegebenen Bedeutungen besitzen und Hal ein Halogenatom darstellt,
. oder mit einer Verbindung der Formel (III/a) 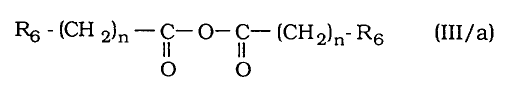
in der R₆ und n die oben angegebenen Bedeutungen besitzen, im Rahmen einer katalytischen Hydrierung umsetzt zur Bildung einer Verbindung der Formel (I/a) 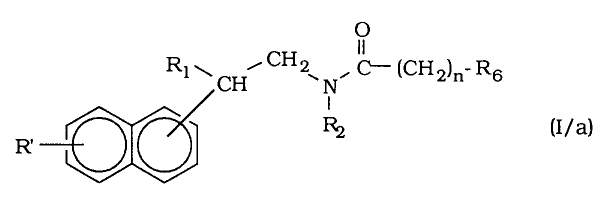 in der R', R₁, R₂, R₆ und n die oben angegebenen Bedeutungen besitzen, einem Sonderfall der Verbindungen der Formel (I) nach Anspruch 1, worin R eine Gruppe R' und R₃ eine Gruppe 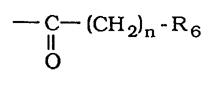 bedeuten,
- oder mit einem Isocyanat oder Isothiocyanat der Formel (IV):
X = C = N - (CH₂)_{n'} - R₇ (IV)
in der X, n' und R₇ die bezüglich der Formel (I) in Anspruch 1 angegebenen Bedeutungen besitzen, umsetzt zur Bildung einer Verbindung der Formel (I/c): 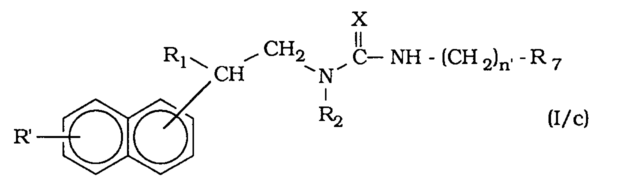 in der R', R₁, R₂, R₇, X und n' die oben angegebenen Bedeutungen besitzen, einem Sonderfall der Verbindungen der Formel (I), in der R eine Gruppe R' und R₃ eine Gruppe 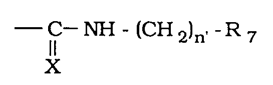 darstellen, wobei die Verbindungen der Formeln (I/a), (I/b) und (I/c) auch, wenn R' eine Hydroxylgruppe darstellt, mit einer Verbindung der Formel (V)
R'' - Hal' (V)
in der R'' eine gegebenenfalls substituierte (wobei der Begriff substituiert die in Anspruch 1 angegebenen Bedeutungen besitzt) Gruppe ausgewählt aus Cycloalkyl-, Cycloalkylalkyl-, Phenyl-, Phenylalkyl- und Diphenylalkylgruppen und Hal' ein Halogenatom bedeuten, umgesetzt werden können zur Bildung einer Verbindung der Formel (I/d) 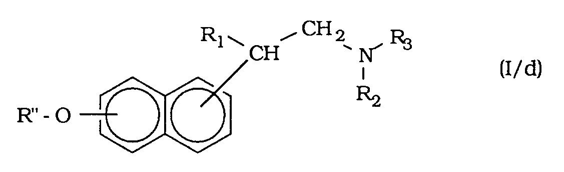 in der R₁, R₂, R₃ und R'' die oben angegebenen Bedeutungen besitzen, einem Sonderfall der Verbindungen der Formel (I) nach Anspruch 1, worin R eine Gruppe -O-R'' darstellt,
wobei die Verbindungen der Formeln (I/a), (I/b), (I/c) und (I/d) gemeinsam die Verbindungen der Formel (I) von Anspruch 1 bilden,
und wobei die Verbindungen der Formel (I) nach Anspruch 1:
- gemäß einer oder mehreren Methoden zur Reinigung ausgewählt aus Kristallisation, Chromatographie über Kieselgel, Extraktion, Filtration und Überführung über Kohlenstoff oder ein Harz gereinigt werden können,
- gegebenenfalls in reiner Form oder in Form einer Mischung in ihre eventuellen optischen Isomeren aufgetrennt werden können,
- oder, wenn R₁ eine Carboxylgruppe darstellt oder R eine Hydroxylgruppe bedeutet, mit einer pharmazeutisch annehmbaren Base in die Salze überführt werden können.

16. Verfahren zur Herstellung der Verbindungen der Formel (I/e) 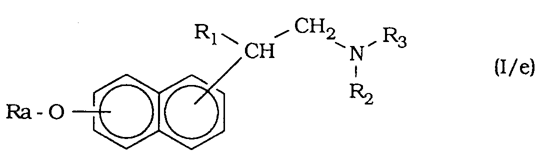 in der R₁, R₂ und R₃ die bezüglich der Formel (I) angegebenen Bedeutungen besitzen und Rₐ eine gesättigte Cycloalkyl- oder Cycloalkylalkyl-gruppe darstellt, einem Sonderfall der Verbindungen der Formel (I) nach Anspruch 1, worin R eine Gruppe -O-Rₐ darstellt,
**dadurch gekennzeichnet**, daß man eine Verbindung der Formel (I/f) 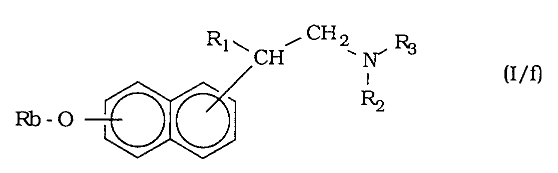 in der R₁, R₂ und R₃ die oben angegebenen Bedeutungen besitzen und R_{b} eine ungesättigte Cycloalkenyl- oder Cycloalkenylalkyl-gruppe darstellt, einem Sonderfall der Verbindungen der Formel (I) nach Anspruch 1, worin R eine Gruppe -O-R_{b} darstellt,
einer katalytischen Hydrierung unterwirft zur Bildung der Verbindung der Formel (I/a), worin Rₐ der gesättigten Form der Gruppe R_{b} entspricht,
welche Verbindungen der Formel (I/e):
- gemäß einer oder mehreren Reinigungsmethoden, ausgewählt aus Kristallisation, Chromatographie über Kieselgel, Extraktion, Filtration und Überführung über Kohlenstoff oder ein Harz gereinigt werden können,
- gegebenenfalls in reiner Form oder in Form einer Mischung in ihre eventuellen optischen Isomeren aufgetrennt werden können.
- oder dann, wenn R₁ eine Carboxylgruppe darstellt, mit einer pharmazeutisch annehmbaren Base in die Salze überführt werden können.

17. Pharmazeutische Zubereitung enthaltend als Wirkstoff mindestens eine der Verbindungen der Formel (I) nach Anspruch 1 in Kombination mit einem oder mehrern inerten, nichttoxischen, pharmazeutisch annehmbaren Trägermaterialien oder Bindemitteln.

18. Pharmazeutische Zubereitung nach Anspruch 17 für die Verwendung bei der Behandlung von Störungen, die mit einer anormalen Aktivität des Melatonins im Organismus verbunden sind, sowie zur Behandlung von Störungen des Zentralnervensystems, des Immunsystems und des endokrinen Systems.
